**Europäisches Patentamt**

**European Patent Office**

**Office européen des brevets**

(19)

(11) Veröffentlichungsnummer: **0 270 868**

**B1**

(12)

# EUROPÄISCHE PATENTSCHRIFT

(45) Veröffentlichungstag der Patentschrift: **30.01.91**

(21) Anmeldenummer: **87116759.9**

(22) Anmeldetag: **30.05.85**

(60) Veröffentlichungsnummer der früheren Anmeldung nach Art. 76 EPÜ: **0 167 783**

(51) Int. Cl.⁵: **B 65 D 47/08, E 05 D 1/02**

(54) **Behälter für diagnostische Testträger.**

(30) Priorität: **28.06.84 DE 3423851**
**22.12.84 DE 3447223**
**16.02.85 DE 3505454**

(43) Veröffentlichungstag der Anmeldung:
**15.06.88 Patentblatt 88/24**

(45) Bekanntmachung des Hinweises auf die Patenterteilung:
**30.01.91 Patentblatt 91/05**

(84) Benannte Vertragsstaaten:
**AT BE CH DE FR GB IT LI NL SE**

(56) Entgegenhaltungen:
**DE-A-1 808 875**
**FR-A-1 574 819**
**FR-A-2 288 683**
**FR-A-2 407 140**
**US-A-3 283 964**
**US-A-3 567 085**
**US-A-3 918 578**

(73) Patentinhaber: **BOEHRINGER MANNHEIM GMBH**
**Sandhofer Strasse 116**
**D-6800 Mannheim 31 (DE)**

(72) Erfinder: **Koehn, Jochen**
**Bergwinkel 4**
**D-5583 Zell/Mosel (DE)**
Erfinder: **Brach, Ulrich**
**Königsbergerweg 10a**
**D-5583 Zell/Mosel (DE)**
Erfinder: **Sacherer, Klaus Dieter**
**Westring 17**
**D-6719 Kirchheim/Weinstrasse (DE)**
Erfinder: **Weiss, Erich**
**Zwerchgasse 49**
**D-6800 Mannheim 31 (DE)**

(74) Vertreter: **Pfeifer, Hans-Peter et al**
**Patentanwälte Dr. Moser und Dr. Pfeifer**
**Nowackanlage 15**
**D-7500 Karlsruhe 1 (DE)**

Courier Press, Leamington Spa, England.

## Beschreibung

Die Erfindung bezieht sich auf einen Testträgerbehälter gemäß dem Oberbegriff des Patentanspruchs 1. Ein solcher Behälter ist bekannt.

Die Reaktionsschicht diagnostischer Testträger ist sehr empfindlich gegen Feuchtigkeit. Gleichwohl soll es möglich sein, die Testträger in dem Behälter bis zu mehreren Jahren zu lagern, ohne daß ihre Funktion beeinträchtigt wird. Der Behälter muß also feuchtigkeitsdicht sein und nach der Entnahme von Testträgern wieder feuchtigkeitsdicht zu verschließen sein. Die Handhabung soll einfach sein, da Testträger auch von Nichtmedizinern verwendet werden, z.B. zur Selbstbestimmung des Blutzuckergehaltes. Erwünscht ist (z.B. von Diabetikern) ein Behälter, der z.B. in einer Jackentasche gut mitzuführen ist, und eine einfache Entnahme der (meist streifenförmigen) Testträger erlaubt.

Der Erfindung liegt die Aufgabe zugrunde, einen Behälter für Testträger zu schaffen, die diese Anforderungen erfüllt und außerdem kostengünstig herzustellen ist. Diese Aufgabe wird durch einen Behälter gemäß Patentanspruch 1 gelöst.

Der abgeflachte Querschnitt erlaubt ein bequemes Unterbringen des Behälters. Die kleine Querschnittsfläche der Entnahmeöffnung erleichtert das Abdichten des Verschlusses, da die Dichtlänge klein ist. Es lassen sich bequem einzelne streifenförmige Testträger entnehmen. Das abnehmbare Bodenelement erlaubt ein schnelles Füllen des Behälters.

Dadurch, daß die Verschließvorrichtung für die Entnahmeöffnung ein von dem Behälterkörper gesondertes Bauteil ist, ist es möglich, auch relativ kompliziert geformte Behälter im Spritzgußverfahren ohne komplizierte Formen herzustellen. Es ist nicht nötig, dabei auf den Behälter oder eine eventuell an dem Behälter vorhandene Überkappe Rücksicht zu nehmen. Da diese getrennt, ebenfalls als Spritzgußteile hergestellt werden können, ist es möglich, sowohl den Behälter wie gegebenenfalls seine Überkappe als auch die Verschließvorrichtung jeweils mit einfachen Formen im Spritzgußverfahren herzustellen. Somit ergibt sich eine kostengünstige Gesamtheit dieser Bestandteile.

Für andere Anwendungszwecke sind die erwähnten technischen Elemente teilweise bekannt. Insbesondere ist in der FR—A—2 288 683 ein Behälter für Flüssigkeiten beschrieben, bei dem das Verschließelement Teil einer vom Behälterkörper getrennten Verschließvorrichtung ist, wobei auch weitere konstruktive Einzelheiten (zwei mit einem Filmscharnier verbundene Schenkel, von denen einer das Schließelement trägt und der andere zur Befestigung an dem Behälterkörper dient, Dichtung mit Hilfe einer an einen Dichtrand der Entnahmeöffnung angepaßten Dichtfläche) übereinstimmen. Ein Hinweis auf die Lösung der spezifischen mit der Verpackung von Testträgern verbundenen Probleme ist dieser Literaturstelle jedoch nicht zu entnehmen. Bei der Verpackung von Flüssigkeiten herrschen sowohl bezüglich des Füllens, als auch bezüglich der Entnahme völlig verschiedene Verhältnisse. Die Anforderungen an die Dichtigkeit, insbesondere gegen das Eindringen von Wasserdampf, sind nicht vergleichbar.

Weitere Beispiele für die Verwendung von Federscharnieren sind der DE—A—1 808 875 und der FR—A—2 407 140 zu entnehmen.

Bevorzugt ist ein Trockenmittel im Bodenelement statt wie bisher üblich im Verschluß untergebracht. Die Lebensdauer des Trockenmittels wird dadurch verlängert, daß es beim Öffnen des Behälters nicht mehr in vollem Maße der Raumfeuchtigkeit ausgesetzt wird.

Nach Anspruch 3 läßt sich eine hohe mechanische Stabilität und Feuchtigkeitsdichtigkeit des Behälters erzielen.

Nach Anspruch 4 läßt sich eine wirkungsvolle Dichtung des Verschlusses bei geringen Kosten realisieren.

Gemäß einer weiteren bevorzugten Ausführungsform ist der Verschlußschenkel und das Befestigungsteil der Verschließeinrichtung über ein Schnappscharnier miteinander verbunden. Ein Schnappscharnier in diesem Sinne ist ein Scharnier, welches innerhalb des Schwenkweges des einen Scharnierteils gegenüber dem anderen eine Stellung labilen Gleichgewichts hat, aus der das Scharnier in eine Offenstellung oder eine Schließstellung schnappt, um in dieser Stellung dann stehen zu bleiben.

Bei Verwendung eines solchen Schnappscharniers wird eine besonders günstige Bauform dadurch erreicht, daß das federnde Verbindungsstück in Schließstellung gegen die Wand des Behälters oder der Überkappe gerichtet ist. Es tritt also bei geschlossenwm Behälter nach außen nicht störend in Erscheinung. Die Außenfläche des Schnappscharniers bildet in Schließstellung z.B. eine Ebene oder eine dem Behälter oder seiner Überkappe angepaßte gewölbte Fläche, die das Hauptgelenk enthält, so daß sich ein befriedigender ästhetischer Eindruck ergibt. Es stehen keine Scharnierteile nach außen störend hervor.

Eine weitere bevorzugte Ausführungsform sieht vor, daß die beiden Scharnierteile plattenförmig ausgebildet sind und die Platten eine ausreichende Dicke haben, so daß sich die Gelenke des Scharniers besonders günstig anordnen lassen.

Im Hinblick auf die Verschlußsicherheit ist es besonders vorteilhaft, wenn das Verschließelement der Verschließeinrichtung nicht ohne weiteres die Entnahmeöffnung beim Zuschnappen völlig abdeckt. Die Kraft eines Schnappscharniers reicht im allgemeinen nämlich nicht aus, eine Entnahmeöffnung auch dicht zu schließen, da hierzu der Widerstand von Dichtwülsten und dergleichen überwunden werden muß. Durch die Maßnahmen gemäß Anspruch 9 wird kenntlich gemacht, daß der Verschluß noch offen ist, so daß der Verbraucher darauf aufmerksam gemacht wird, daß er den Verschluß noch zudrük-

ken muß.

Die Erfindung wird im folgenden anhand eines in den Figuren schematisch dargestellten Ausführungsbeispiels näher erläutert. Es zeigen:

Figur 1 eine Ansicht einer ersten Ausführungsform einer mit einem Schnappscharnier ausgerüsteten Verschließvorrichtung für einen erfindungsgemäßen Behälter von unten,

Figur 2 eine Seitenansicht derselben Verschließvorrichtung,

Figur 3 einen mittigen Längsschnitt durch diese Verschließvorrichtung,

Figur 4 einen mittigen Längsschnitt durch den oberen Teil eines zugehörigen Behälters,

Figur 5 die Verschließvorrichtung in Seitenansicht nach Figur 2, jedoch geöffnet,

Figur 6 perspektivisch einen erfindungsgemäßen Behälter zur Aufnahme von Testträgern in seiner Gesamtheit,

Figur 7 perspektivisch ein zugehöriges Bodenelement,

Figur 8 einen Längsschnitt durch Behälter und Bodenelement nach Figur 6 und 7.

In allen Ausführungsbeispielen beziehen sich Begriffe wie "oben", "unten" und dergleichen immer auf die Lage einer Verschließvorrichtung an einem aufrechtstehenden Behälter, bei dem die Mündung aufwärts gerichtet ist oder an einer auf den Behälter gesteckten Überkappe.

Alle dargestellten Verschließvorrichtungen werden einteilig, z.B. aus Polypropylen gespritzt.

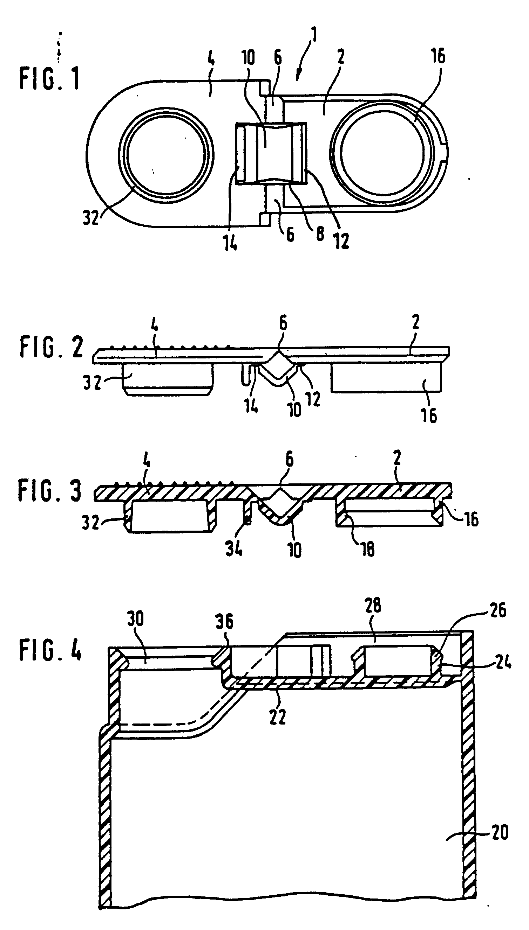

Die in den Figuren 1 bis 3 und 5 dargestellte Verschließvorrichtung 1 ist besonders für einen erfindungsgemäßen Testträgerbehälter geeignet. Sie hat zwei plattenartige Bestandteile, nämlich einen Befestigungsteil 2 und einen Verschlußschenkel 4, die durch ein als Filmscharnier ausgebildetes Hauptgelenk 6 miteinander verbunden sind. Das Hauptgelenk ist zweiteilig ausgebildet. Zwischen seinen beiden Teilen befindet sich eine Öffnung 8, in der ein federndes Verbindungsstück 10 untergebracht ist. Das Verbindungsstück 10 steht durch zwei Nebengelenke 12 und 14 mit dem Befestigungsteil 2 und dem Verschlußschenkel 4 in Verbindung. Der Befestigungsteil 2 trägt eine Steckhülse 16, die mit einem Innenwulst 18 versehen ist.

Figur 4 zeigt das Oberende eines erfindungsgemäßen Behälters 20. Auf dessen oberer Wand befindet sich eine Steckhülse 24 mit einem Außenwulst 26. Der Befestigungsteil 2 läßt sich mit Hilfe seiner Steckhülse 16 auf der Steckhülse 24 des Behälters verankern. Ein die Wand 22 umlaufender Wandteil 28 des Behälters sorgt für eine gleichbleibende Orientierung der Verschließvorrichtung 1.

Der Behälter hat links eine Entnahmeöffnung 30. An dem Verschlußschenkel 4 ist ein dazu passendes Verschließelement 32 in Form eines Rohrstutzens vorgesehen. Am Verschlußschenkel 4 befindet sich ein Vorsprung 34 von der Breite des federnden Verbindungsstücks 10. Der Vorsprung ist so angeordnet, daß er beim Herunterschnappen des Verschlußschenkels 4 einen zugehörigen Anschlag 36, nämlich den Rand der Entnahmeöffnung 30, streifend berührt. Der Verschlußschenkel bleibt auf diese Weise zunächst offen stehen, läßt sich dann aber unter Überwindung des Widerstandes zwischen Vorsprung 34 und Anschlag 36 schließen.

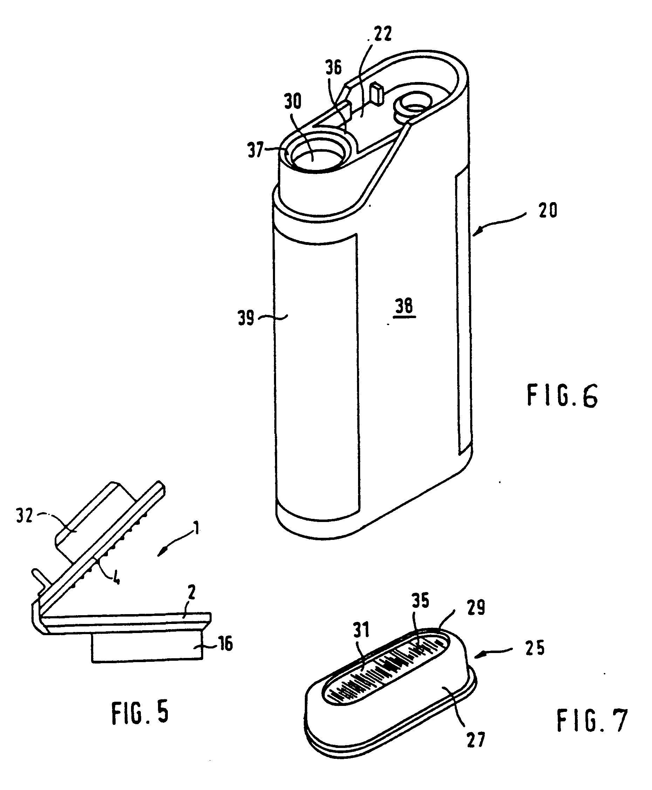

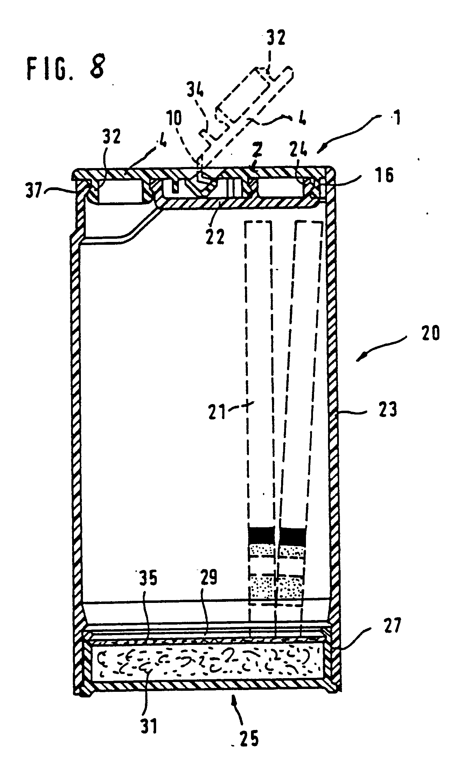

Figuren 6 bis 8 zeigen einen erfindungsgemäßen Behälter 20 für eine Vielzahl von Testträgern 21, von denen nur zwei dargestellt sind. Der Behälter hat einen Behälterkörper 23 und eine Verschließvorrichtung 1, wie sie in den Figuren 1 bis 3 dargestellt ist.

In das untere, offene Ende des Behälterkörpers 23 ist ein Bodenelement 25 einsteckbar. Die Innenwandung des unteren Endes des Behälterkörpers und die Außenwandung 27 des Bodenelementes sind so einander angepaßt, daß das Bodenelement feuchtigkeitsdicht in den Behälterkörper einsteckbar ist. Die Außenwandung des Bodenelementes kann mit der Innenwandung des unteren Endes des Behälterkörpers durch Ultraschallschweissung luftdicht und damit auch feuchtigkeitsdicht verbunden werden.

Das Bodenelement umschließt bis auf eine Einfüllöffnung 29 eine Kammer 31, die zur Aufnahme eines Trockenmittels dient. Die Kammer ist gegen den Innenraum des Behälters 20 mit einem die Feuchtigkeitsaufnahme regulierenden Element, beispielsweise einer Kartonscheibe 35 verschlossen.

Wie Figur 6 zeigt, ist der Behälter 20 flach. Er hat parallele Seitenwände 38 und abgerundete Endseiten 39. Er könnte auch einen elliptischen Querschnitt haben. Die Entnahmeöffnung 30 ist etwa konzentrisch zu der Rundung der einen Endseite angeordnet. Hierdurch wird die Entnahme von Testträgern vereinfacht. Die Packung braucht hierzu nur in Richtung der Entnahmeöffnung gekippt werden.

Ein zu diesem Behälter passender Verschluß ist in den Figuren 1 bis 3 dargestellt und wurde oben beschrieben. Die Deckwand 22, die die obere Endfläche des Behälterkörpers darstellt, ist ein integraler Bestandteil des Behälterkörpers. Die einzige Öffnung des Behälterkörpers nach oben ist die kreisrunde Entnahmeöffnung 30. Diese läßt sich durch das Verschließelement 32, das am Verschlußschenkel 4 sitzt, verschließen. Das Verschließelement 32 hat die Form eines Ringes, dessen Außenwandung im Querschnitt kreisförmig ist. Diese Außenwandung bildet die Dichtfläche, die im Schließzustand dichtend an einem Innenwulst 37 der Entnahmeöffnung 30 anliegt. Die obere Wandung dieses Innenwulstes verläuft konisch. Sie verjüngt sich gegen das Behälterinnere. Der Innenwulst und die ihn umgebenden Wandteile sind gemeinsam relativ dick und geben beim Verschließen nur wenig nach. Dagegen ist das Verschließelement 32 verhältnismäßig dünnwandig und wird beim Schließen geringfügig gegen das Zentrum der Öffnung zusammengedrückt. Die kreisrunde Form der Entnahmeöffnung 30 und des Verschließelementes 32 führen hier zu einem günstigen Ringspannverhalten und damit zu einer guten Abdichtung.

Die Funktion des Vorsprunges 34 des Verschlußschenkels 4 wurde schon an Hand der Figuren 2—4 beschrieben. Es ist wichtig, daß ein Behälter mit feuchtigkeitsempfindlichen Inhalt stets vollständig geschlossen wird. Durch den Anschlag des Vorsprunges 34 an der Berandung der Entnahmeöffnung (Anschlag 36) wird dem Benutzer kenntlich gemacht, daß der Behälter nicht geschlossen ist, was weniger deutlich zu erkennen wäre, wenn der Verschlußschenkel 4 weiter zuklappen würde. Der Benutzer wird also genötigt, den Verschlußschenkel vollständig niederzudrücken.

Ergänzend wird auf den Inhalt der europäischen Patentanmeldung 85 106 641.5 (Publikationsnummer 167 783) Bezug genommen, zu der die vorliegende Anmeldung eine Teilanmeldung ist.

## Patentansprüche

1. Testträgerbehälter für streifenförmige diagnostische Testträger mit

einem länglichen Behälterkörper (23) aus Kunststoff mit einem Entnahmeende, an dem sich eine Entnahmeöffnung (30) befindet und einem Boden an seinem entgegengesetzten Ende,

einer am Entnahmeende vorgesehenen Verschließvorrichtung (1) für die Entnahmeöffnung (30) und

einer Trockenmittelkammer

dadurch gekennzeichnet, daß

die Verschlußvorrichtung ein von dem Behälterkörper gesondertes Bauteil ist, das ein Befestigungsteil (2) und einen Verschlußschenkel (4) aufweist, wobei das Befestigungsteil (2) zum dauerhaften Befestigen an einer Wand, insbesondere oberen Abschlußwand (22) des Behälters dient und der Verschlußschenkel (4) ein zur Entnahmeöffnung (30) passendes Verschließelement (32) mit einer an einen Dichtrand der Entnahmeöffnung angepaßten Dichtfläche hat,

der Behälterkörper (23) in einer Ebene quer zu seiner Längsrichtung einen abgeflachten Querschnitt aufweist,

die Querschnittsfläche der Entnahmeöffnung (30) groß genug zur Entnahme der Testträger aber erheblich kleiner als die Querschnittsfläche des Behälterkörpers (23) ist und

ein zum Füllen des Behälters mit Testträgern abnehmbares Bodenelement (25) vorgesehen ist, welches in den Behälterkörper (23) so eingesetzt und mit ihm verbunden wird, daß es den Behälterkörper (23) feuchtigkeitsdicht verschließt.

2. Behälter nach Anspruch 1, gekennzeichnet durch folgende Merkmale:

a) das Bodenelement (25) hat eine Kammer für ein Trockenmittel,

b) die Kammer ist gegen das Innere des Behälters (20) durch ein die Flüssigkeitszufuhr regulierendes Element (35) verschlossen.

3. Behälter nach Anspruch 1 oder 2, dadurch gekennzeichnet, daß Behälterkörper (23) und Bodenelement (25) eine Wandstärke von mindestens etwa 1 mm haben und aus Polyethylen oder Polypropylen bestehen.

4. Behälter nach Anspruch 2 oder 3, dadurch gekennzeichnet, daß das Bodenelement (25) mit der zugehörigen Öffnung des Behälterkörpers durch Ultraschallschweißung luft- und feuchtigkeitsdicht verbunden sind.

5. Behälter nach Anspruch 1, gekennzeichnet durch folgende Merkmale:

a) das Verschließelement (32) ist ein ringförmiger Vorsprung der vom Verschlußschenkel senkrecht vorsteht,

b) der Vorsprung ist gegen sein Zentrum elastisch komprimierbar,

c) die Dichtfläche hat einen kreisförmigen Querschnitt und ist so ausgebildet, daß sie beim Verschließen weniger nachgibt als der Vorsprung.

6. Behälter nach einem der Ansprüche 1—5, dadurch gekennzeichnet, daß der Behälterkörper (23) an dem Entnahmeende mindestens eine abgerundete Endseite (39) hat und die Entnahmeöffnung (30) etwa konzentrisch zu der Rundung der Endseite (39) ist.

7. Behälter nach einem der Ansprüche 1—5, gekennzeichnet durch folgende Merkmale:

a) der Verschlußschenkel (4) und das Befestigungsteil (2) der Verschließeinrichtung (1) sind über ein Schnappscharnier verbunden, bei dem die beiden Scharnierteile durch ein Hauptgelenk (6) miteinanderverbunden sind,

b) mindestens ein bogen- oder winkelförmiges, federndes Verbindungsstück (10) ist an seinen beiden Enden durch Nebengelenke (12, 14) mit beiden Scharnierteilen verbunden,

c) die Nebengelenke haben quer zu den Gelenkachsen je einen Abstand vom Hauptgelenk,

d) Hauptgelenk und Nebengelenke sind als Filmscharniere ausgebildet,

e) derart, daß die Scharnierteile die Tendenz haben, aus einer Stellung labilen Gleichgewichts, die sich innerhalb ihres Schwenkbereiches befindet, in die eine oder andere von zwei Endstellungen zu schnappen.

8. Behälter nach Anspruch 7, gekennzeichnet durch die folgenden Merkmale:

f) das federnde Verbindungsstück (10) weist in Schließstellung der an der Wand angebrachten Verschließvorrichtung gegen die Wand,

g) das Hauptgelenk (6) ist an der von der Wand abgekehrten Seite des Schnappscharniers angeordnet.

9. Behälter nach Anspruch 7 oder 8, gekennzeichnet durch folgende Merkmale:

a) der Verschlußschenkel (4) und der Befestigungsteil (2) sind bis auf vorspringende Abschnitte wie Verstärkungsleisten, Gelenkteile und das Verschließelement plattenartig ausgebildet,

b) das Hauptgelenk (6) einerseits und die Nebengelenke (12, 14) andererseits befinden sich an einander gegenüberliegenden Flächen von Verschlußschenkel und Befestigungsteil.

10. Behälter nach einem der Ansprüche 1 bis 9, dadurch gekennzeichnet, daß der Verschlußschenkel (4) einen Vorsprung (34) aufweist, der so angeordnet ist, daß er beim Schließen der Entnahmeöffnung einen zugeordneten Anschlag (36) am Behälter oder dem gesonderten Bauteil streifend berührt und dadurch einem Fortsetzen der Schließbewegung einen spürbaren Widerstand entgegensetzt.

**Revendications**

1. Récipient de support de test pour support de test en forme de bande, destiné au diagnostic, comprenant

un corps de récipient (23) longitudinal en matière plastique, comprenant une extrémité de prélèvement, sur laquelle se trouve un orifice de prélèvement (30), et un fond à son extrémité opposée,

un dispositif d'obturation (1) prévu à l'extrémité de prélèvement pour l'orifice de prélèvement (30) et

une chambre pour un agent de séchage, caractérisé en ce que le dispositif d'obturation est un composant particulier du corps de récipient, qui présente une pièce de fixation (2) et un volet d'obturation (4), la pièce de fixation (2) servant à la fixation permanente sur une paroi, en particulier la paroi terminale supérieure (22) du récipient, et le volet d'obturation (4) comprenant un élément d'obturation (32) ajusté sur l'orifice de prélèvement (30), ayant une face d'étanchéité ajustée au bord d'étanchéité de l'orifice de prélèvement,

le corps de récipient (23) présente, dans un plan perpendiculaire à sa direction longitudinale, une section transversale aplatie,

la face de section transversale de l'orifice de prélèvement (30) est suffisamment grande pour le prélèvement du support de test, mais nettement plus petite que la face de section transversale du corps de récipient (23), et

il est prévu un élément de fond (25), pouvant être enlevé pour l'introduction du support de test dans le récipient, qui est placé dans le corps de récipient (23) et relié à celui-ci de façon à fermer le corps de récipient (23) de manière étanche à l'humidité.

2. Récipient selon la revendication 1, caractérisé en ce que:

a) l'élément de fond (25) présente une chambre pour un agent de séchage,

b) la chambre est fermée par rapport à l'intérieur du récipient (20) au moyen d'un élément (35) régulant l'amenée de liquide.

3. Récipient selon la revendication 1 ou 2, caractérisé en ce que le corps de récipient (23) et l'élément de fond (25) présentent une épaisseur de paroi d'au moins 1 mm et sont constitués de polyéthylène ou de polystyrène.

4. Récipient selon la revendication 2 ou 3, caractérisé en ce que l'élément de fond (25) est relié à l'orifice correspondant du corps de récipient par l'intermédiaire d'une soudure par ultrasons, de manière étanche à l'air et aux liquides.

5. Récipient selon la revendication 1, caractérisé en ce que:

a) l'élément d'obturation (32) est une protubérance de forme annulaire qui fait saillie perpendiculairement au volet d'obturation,

b) la protubérance peut subir une compression élastique en direction de son centre,

c) la face d'étanchéité présente une section transversale circulaire et est conçue de telle façon, que lors de l'obturation, elle fléchit moins que la saillie.

6. Récipient selon l'une quelconque des revendications 1 à 5, caractérisé en ce que le corps de récipient (23) présente, à l'extrémité de prélèvement, au moins une face d'extrémité (39) arrondie, et que l'orifice de prélèvement (30) est sensiblement concentrique par rapport à l'arrondi de la face d'extrémité (39).

7. Récipient selon l'une quelconque des revendications 1 à 5, caractérisé en ce que:

a) le volet d'obturation (4) et la pièce de fixation (2) du dispositif d'obturation (1) sont reliés entre eux par une charnière à ressort, les deux parties de la charnière étant reliées entre elles par une articulation principale (6),

b) au moins une pièce de liaison (10) résiliente, en forme d'arc ou d'angle, est reliée, à ses deux extrémités, par des articulations secondaires (12, 14), aux deux parties de la charnière,

c) les articulations secondaires sont éloignées chacune de l'articulation principale suivant une direction perpendiculaire à l'axe d'articulation,

d) l'articulation principale et les articulations secondaires sont conçues sous forme de filmcharnière,

e) de telle façon, que les parties de charnière ont tendance à se rabattre, depuis une position d'équilibre instable, qui se trouve à l'intérieur de leur domaine de pivotement, dans l'une ou l'autre des deux positions d'extrémité.

8. Récipient selon la revendication 7, caractérisé en ce que:

f) la pièce de liaison (10) résiliente est dirigée, dans la position d'obturation du dispositif d'obturation monté sur la paroi, vers la paroi,

g) l'articulation principale (6) est disposée sur la face de la charnière rabattante opposée à la paroi.

9. Récipient selon la revendication 7 ou 8, caractérisé en ce que:

a) le volet d'obturation (4) et la pièce de fixation (2) sont conçus sous forme de plaques, sauf les segments en saillie tels que les bandeaux de renforcement, les pièces d'articulation et l'élément d'obturation,

b) l'articulation principale (6) d'une part et les articulations secondaires (12, 14) d'autre part se trouvent sur des faces opposées du volet d'obturation et de la pièce de fixation.

10. Récipient selon l'une quelconque des revendications 1 à 9, caractérisé en ce que le volet d'obturation (4) présente une protubérance (34) qui est disposée de telle façon que lors de l'obturation de l'orifice de prélèvement, une butée (36) associée entre en contact rasant avec le récipient ou le composant particulier, et de ce fait, oppose une résistance sensible à la poursuite du mouvement d'obturation.

## Claims

1. Test carrier container for strip-shaped diagnostic test carriers with a longitudinal container body (23) of synthetic material with a removal end on which is present a removal opening (30) and a bottom on its opposite end, a closure device (1) for the removal opening (30) provided on the removal end and a drying agent chamber, characterised in that the closure device is a constructional part separate from the container body, which has a fixing part (2) and a closure limb (4), whereby the fixing part (2) serves for the permanent fixing on a wall, especially upper closure wall (22) of the container and the closure limb (4) has a closure element (32), adapted to the removal opening (30), with a sealing surface adapted to a sealing edge of the removal opening, the container body (23) has a flattened cross-section in a plane transverse to its longitudinal direction, the cross-sectional surface of the removal opening (30) is large enough for the removal of the test carrier but considerably smaller than the cross-sectional surface of the container body (23) and a removable bottom element (25) is provided for the filling of the container with test carriers which is so inserted in the container body (23) and connected therewith that it moisture-tightly closes the container body (23).

2. Container according to claim 1, characterised by the following features:

a) the bottom element (25) has a chamber for a drying agent,

b) the chamber is closed against the interior of the container (20) by an element (35) regulating the liquid inlet.

3. Container according to claim 1 or 2, characterised in that container body (23) and bottom element (25) have a wall thickness of at least about 1 mm and consist of polyethylene or polypropylene.

4 Container according to claim 2 or 3 characterised in that the bottom element (25) is air- and moist-tightly connected with the associated opening of the container body by ultrasonic welding.

5. Container according to claim 1, characterised by the following features:

a) the closure element (32) is a ring-shaped projection which projects vertically from the closure limb,

b) the projection is flexibly compressible towards its centre,

c) the sealing surface has a circular-shaped cross-section and is so formed that, in the case of closing, it gives less than the projection.

6. Container according to one of claims 1—5, characterised in that the container body (23) has, on the removal end, at least one rounded end side (39) and the removal opening (30) is approximately concentric to the rounding of the end side (39).

7. Container according to one of claims 1 5, characterised by the following features:

a) the closure limb (4) and the fixing part (2) of the closure device (1) are connected via a snap hinge in which the two hinge parts are connected with one another by a main joint (6),

b) at least an arc- or angle-shaped, flexible connecting piece (10) is connected on its two ends by subsidiary joints (12, 14) with both hinge parts,

c) the side joints each have, transversely to the joint axes, a distance from the main joint,

d) main joint and side joints are formed as film hinges,

e) in such a manner that the hinge parts have the tendency to snap from a position of labile equilibrium, which is present within its tilting range, into one or other of two end positions.

8. Container according to claim 7, characterised by the following features:

f) the flexible connection piece (10) points towards the wall in the closure position of the closure device positioned on the wall,

g) the main joint (6) is arranged on the side of the snap hinge facing away from the wall.

9. Container according to claim 7 or 8, characterised by the following features:

a) the closure limb (4) and the fixing part (2) are, apart from projecting sections, such as reinforcement bars, joint parts and the closure element, formed plate-like,

b) the main joint (6), on the one hand, and the side joints (12, 14), on the other hand, are present on surfaces of closure limb and fixing part lying opposite one another.

10. Container according to one of claims 1 to 9, characterised in that the closure limb (4) has a projection (34) which is so arranged that, in the case of closure of the removal opening, it glancingly touches an associated stop (36) on the container or the separate constructional part and thereby encounters a detectable resistance to a continuation of the closing movement.

FIG. 1

FIG. 2

FIG. 3

FIG. 4

FIG.6

FIG.5

FIG.7

FIG. 8